# EUROPEAN PATENT SPECIFICATION

(11) **EP 1 147 086 B1**
(45) Date of publication and mention of the grant of the patent: **30.03.2005**
(21) Application number: 00900030.8
(22) Date of filing: 07.01.2000
(51) Int. Cl.: C07D 213/80, C07D 213/82, A61K 31/4406, A61K 31/4425, A61K 31/455, A61P 17/02, A61P 17/14

(54) **COMPOSITIONS FOR THE TREATMENT OF SKIN DISEASES**
ZUSAMMENSETZUNGEN ZUR BEHANDLUNG VON HAUTKRANKHEITEN
COMPOSITIONS DESTINEES AU TRAITEMENT DES MALADIES DE LA PEAU

(30) Priority: 07.01.1999 PL 33076899
(43) Date of publication of application: 24.10.2001
(73) Proprietor: Technical University of Lodz, 90-924 Lodz (PL); Gebicki, Jerzy, 94-209 Lodz (PL); Sysa-Jedrzejowska, Anna, 90-138 Lodz (PL); Adamus, Jan, 94-047 Lodz (PL)
(72) Inventor: GEBICKI, Jerzy, PL-94-209 Lodz (PL); SYSA-JEDRZEJOWSKA, Anna, PL-90-138 Lodz (PL); ADAMUS, Jan, PL-94-047 Lodz (PL)
(74) Representative: König, Beate, Dipl.-Phys. Dr.
(86) International application number: PCT/IB2000/000019
(87) International publication number: WO 2000/040559

(56) References cited:
- EP-A- 0 052 705
- EP-A- 0 518 352
- WO-A-98/52927
- DE-A- 3 603 601
- US-A- 3 823 076
- US-A- 4 067 975
- US-A- 4 578 394
- US-A- 4 786 647
- CHEMICAL ABSTRACTS, vol. 77, no. 3, 17 July 1972 (1972-07-17) Columbus, Ohio, US; abstract no. 14772e, page 106; XP002133576 & Y. YABUHARA ET AL.: HAKKO KOGAKU ZASSHI, vol. 50, no. 2, 1972, pages 86-92,

## Description

The present invention relates to methods and compositions for the treatment of skin diseases and disorders. In particular it relates to methods and compositions for the treatment of skin diseases and disorders in which oedema, erythema, cutaneous eruption, dilation of superficial blood vessels and desquamation are manifested (including when accompanied by pruritus and burning sensation), as well as in cases of intensified seborrhoea.

There are several topical therapeutic products in use, mostly containing corticosteroids, which exert anti-inflammatory, oedema-reducing, anti-seborrhoeic and anti-pruritic effects. Nicotinamide, i.e. vitamin PP is also administered in adjunctive medical treatment of skin diseases. There is also an ointment in use, known as "Dernilan", which contains nicotinamide, allantoin, salicylic acid and camphorae which exerts anti-inflammatory and exfoliating effects on the skin. The use of nicotinamide for the treatment of acne vulgaris is also disclosed in EP-A-0052705; the use of nicotinamide derivatives for the treatment of psoriasis is disclosed in US-A-4,067,975; and the use of certain nicotinamide and nicotinic acid derivatives for the treatment of various skin conditions is disclosed in WO-A-98/52927.

It has now, surprisingly, been found that 1-alkylnicotinamide salts and 1-alkylnicotinic acid ester salts can be used to treat a wide variety of skin diseases and disorders and that the use of these compounds provides certain advantages over the use of nicotinamide, in particular an increased efficacy at a specified dose and/or a reduction in undesirable side effects. In particular, topical treatment of skin diseases such as acnes by administering a solution of the 1-methylnicotinamide salts and 1-methylnicotinic acid ester salts of the present invention has been shown to produce at least a similar therapeutic effect at a concentration approximately 100 times lower than the corresponding treatment with nicotinamide or nicotinic acid respectively, but with no appreciable side effects.

Thus, according to one aspect of the present invention, there is provided the use of a compound of formula (I): 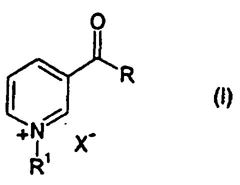 wherein R represents the group NR²R³ or the group OR⁴;
R¹ represents C₁₋₄ alkyl;
R² and R⁴ each independently represent hydrogen or C₁₋₄ alkyl;
R³ represents hydrogen, C₁₋₄ alkyl or CH₂OH;
and X⁻ is a physiologically suitable counter-anion;
in the preparation of a medicament for the treatment of skin diseases or disorders, in particular hair loss, sunburn, burns, scalds and for wound healing.

According to a further aspect of the present invention, there is provided a method of cosmetic treatment of a skin in a human or animal subject, comprising the administration to said subject of an effective amount of a compound of formula (I) as defined above.

According to a further aspect of the present invention, there is provided compound of formula (I) as defined above for use in cosmetology.

In the compounds of formula (I), R² preferably represents ethyl, methyl or hydrogen, more preferably methyl or hydrogen, most preferably hydrogen. Preferably R³ represents CH₂OH, methyl or hydrogen, more preferably CH₂OH or hydrogen, most preferably hydrogen. In a further preferment, R⁴ represents C₁₋₄ alkyl. In a further preferment R⁴ represents propyl, ethyl, methyl or hydrogen, more preferably propyl, ethyl or methyl, most preferably propyl or ethyl. Preferably R represents the group NR²R³.

In an especially preferred embodiment of the present invention, the compound of formula (I) is a 1-methylnicotinamide salt, i.e. R¹ represents methyl, and R² and R³ each represent hydrogen.

In a further especially preferred embodiment of the present invention, the compound of formula (I) is a 1-methyl-N'-hydroxymethylnicotinamide salt, i.e. R¹ represents methyl, R² represents hydrogen, and R³ represents CH₂OH.

In a further especially preferred embodiment of the present invention, the compound of formula (I) is a 1-methylnicotinic acid salt, i.e. R¹ represents methyl, and R⁴ represents hydrogen.

In a further especially preferred embodiment of the present invention, the compound of formula (I) is a 1-methylnicotinic acid ethyl ester or 1-methylnicotinic acid propyl ester salt, i.e. R¹ represents methyl, and R⁴ represents propyl or ethyl.

As indicated above, X⁻ is any physiologically suitable counter-anion. The 1-alkylnicotinamide, 1-alkylnicotinic acid and 1-alkylnicotinic acid ester salts of the present invention can thus be derived from any physiologically acceptable acid, whether organic or inorganic in origin. Suitable inorganic acid salts include, for example, chloride, bromide, iodide and carbonate; suitable organic acid salts include mono-, di- and tri- C₁₋₁₈ carboxylic acid salts, for example, acetate, benzoate, salicylate, glycolate, lactate, maleate and citrate. Preferred salts include chloride, benzoate, salicylate, acetate, citrate and lactate. Especially preferred are chloride salts.

Some of the compounds of formula (I) are commercially available, for example 1-methylnicotinamide chloride (Sigma) and 1-methylnicotinic acid chloride (Sigma). Alternatively, the compounds can be readily prepared from commercially available compounds (including nicotinamide and nicotinic acid) by synthetic methods well-known to the person skilled in the art Such methods would include synthesis from appropriately substituted pyridine compounds.

According to a further aspect of the present invention, there is provided a pharmaceutical formulation comprising a compound of formula (I) together with one or more pharmaceutically acceptable carriers, diluents or excipients.

According to a further aspect of the present invention there is provided a compound of formula (I) 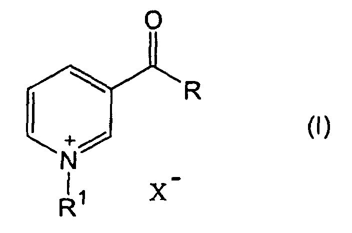 wherein R represents the group NR²R³ or the group OR⁴;
R¹ represents methyl;
R² represents hydrogen or C₁₋₄-alkyl;
R³ represents hydrogen, C₁₋₄-alkyl or CH₂OH;
R⁴ represents C₁₋₄-alkyl;
and X⁻ is physiologically suitable counter-anion;
for use in therapy.

In the treatment of skin conditions, it is possible to administer the compound of formula (I) orally in suitable formulations, preferably tablets or capsules. However, of particular utility are topical formulations of a compound of formula (I). The type of carrier utilised in the present invention depends on the type of product form desired for the composition. The topical compositions useful in the present invention may be made into a wide variety of product forms such as are known in the art. These include, but are not limited to, lotions, creams, gels, sticks, shampoos, soaps,. sprays, ointments, pastes and mousses. These product forms may comprise several types of carriers including, but not limited to, solutions, aerosols, emulsions, gels, solids, and liposomes. Topical formulations are most suitably in the form of an ointment, gel, cream, shampoo, soap, spray, lotion or a solution.

The compound of formula (I) may be administered topically to the skin (including the scalp), or to the mucosal surfaces, for example by intranasal, oral, intravaginal or intrarectal administration. Preferred is topical administration to the skin at the location of the principal manifestation of the skin disease or disorder, the burn or wound.

The topical formulations of the present invention comprise a safe and effective amount of a dermatologically acceptable carrier within which the compound of formula (I) and other optional components are incorporated to enable the compound of formula (I) and other optional components to be delivered to the skin or other relevant site at an appropriate concentration. The carrier can thus act as a diluent, dispersant, solvent, or the like which ensures that the formulation can be applied to and distributed evenly over the selected target to provide an appropriate concentration of the compound of formula (I).

Preferred topical formulations according to the present invention comprise about 90 to 99.95% of a pharmaceutical base carrier and about 0.005 to about 10% by weight of a compound of formula (I) as defined above More preferably the topical formulation contains about 0.01 to about 10% by weight of a compound of formula (I). Preferred pharmaceutical base carriers are an ointment, gel, or aqueous solution. In an ointment the compound of
formula (I) is preferably present at a concentration by weight of 0.1 to 10%, more preferably 0.5 to 10%. In a gel the compound of formula (I) is preferably present in a concentration by weight of 0.05 to 2%, more preferably 0.05 to 1%, most preferably 0.1 to 0.5%. In a solution, the compound of formula (I) is preferably present in a concentration by weight of 0.005 to 0.1%, more preferably 0.005 to 0.05%, most preferably 0.01%.

The carrier may contain one or more dermatologically acceptable solid, semi-solid or liquid fillers, diluents, solvents, extenders and the like. The carrier may be solid, semi-solid or liquid. Preferred carriers are substantially liquid. The carrier can itself be inert or it can possess dermatological benefits of its own. Concentrations of the carrier can vary with the carrier selected and the intended concentrations of the compound of formula (I) and the other optional components.

Suitable carriers include conventional or otherwise known carriers that are dermatologically acceptable. The carrier should also be physically and chemically compatible with the compound of formula (I), and should not unduly impair stability, efficacy or other benefits associated with the formulations of the present invention. Preferred components of the formulations of the present invention should be capable of being comingled in a manner such that there is no interaction which would substantially reduce the efficacy of the formulation under ordinary use situations.

Preferred carriers contain a dermatologically acceptable, hydrophilic diluent As used herein, "diluent" includes materials in which the compound of formula (I) can be dispersed, dissolved, or otherwise incorporated. Nonlimiting examples of hydrophilic diluents are water, organic hydrophilic diluents such as lower monovalent alcohols (e.g., C₁-C₄) and low molecular weight glycols and polyols, including propylene glycol, polyethylene glycol (e.g., Molecular Weight 200-600 g/mole), polypropylene glycol (e.g. Molecular Weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof. Water is a preferred diluent. The composition preferably comprises from about 60% to about 99.99% of the hydrophilic diluent

Solutions according to the subject invention typically include a dermatologically acceptable hydrophilic diluent. Solutions useful in the subject invention preferably contain from about 60% to about 99.99% of the hydrophilic diluent.

Aerosols according to the subject invention can be formed by adding a propellant to a solution such as described above. Exemplary propellants include chloro-fluorinated lower molecular weight hydrocarbons. Additional propellants that are useful herein are described in Sagarin, Cosmetics Science and Technology, 2^{nd} Edition, Vol. 2, pp. 443-465 (1972), incorporated herein by reference. Aerosols are typically applied to the skin as a spray-on product

The topical compositions of the subject invention, including but not limited to lotions and creams, may comprise a dermatologically acceptable emollient Such compositions preferably contain from about 2% to about 50% of the emollient. Emollients tend to lubricate the skin, increase the smoothness and suppleness of the skin, prevent or relieve dryness of the skin, and/or protect the skin. Emollients are typically water-immiscible, oily or waxy materials. A wide variety of suitable emollients are known and may be used herein. Sagarin, Cosmetics Science and Technology, 2nd Edition, Vol.1, pp. 32-43 (1972), incorporated herein by reference, contains numerous examples of materials suitable as an emollient

Lotions and creams according to the present invention generally comprise a solution carrier system and one or more emollients. Lotions typically comprise from about 1% to about 20%, preferably from about 5% to about 10%, of emollient; from about 50% to about 90%, preferably from about 60% to about 80%, water. A cream typically comprises from about 5% to about 50%, preferably from about 10% to about 20%, of emollient; and from about 45% to about 85%, preferably from about 50% to about 75%, water.

Ointments of the present invention may comprise a simple carrier base of animal or vegetable oils or semi-solid hydro-carbons (oleaginous); absorption ointment bases which absorb water to form emulsions; or water soluble carriers, e.g., a water soluble solution carrier. Ointments may further comprise a thickening agent, such as described in Sagarin, Cosmetics, Science and Technology, 2nd edition, Vol. 1, pp. 72-73 (1972), incorporated herein by reference, and/or an emollient. For example, an ointment may comprise from about 2% to about 10% of an emollient; and from about 0.1% to about 2% of a thickening agent.

Preferred ointments comprise Eucerine and glycerol; preferred gels comprise methylcellulose, glycerol and water, or comprise polyacrylic acid, polyethylene glycol, ethanol, triethanolamine, paraben and water; preferred solutions comprise aqueous solutions or solutions of ethyl alcohol or propylene glycol.

In accordance with the invention, the compounds of formula (I) are useful for the treatment of skin disorders or diseases, including crural ulceration, acne juvenile, acne rosacea, psoriasis, atopic dermatitis and vitiligo.

In addition, the compounds of formula (I) have been shown to be useful in the treatment of hair loss, especially alopecia areata, androgenic alopecia, and alopecia caused as a side effect of chemotherapy or radiotherapy.

In addition, the compounds of formula (I) have been shown to be of use in the treatment of burns and scalds (particularly first and first/second degree burns and scalds) and in wound healing, as well as in treating sunburn.

A prophylactic or preventative capability of the compounds of formula (I) has also been shown for chronic and recurrent diseases such as, for example, psoriasis, leg ulcers or acne. The preventive effect consists in keeping the improvement of a previously healed status for a prolonged period of time after cessation of active pathological symptoms.

The compounds of formula (I) also show utility in cosmetology, in particular in providing regeneration and smoothing of the skin; thus treatment of skin ageing effects, such as wrinkles etc. is also contemplated by the present invention.

Additional excipients well known in the art can also be included in the formulations of the present invention; in particular, commonly used stabilisers may be advantageously included to allow the formulations to remain stable for a suitable period, for example, for up to 2 years.

According to a further aspect of the present invention there is also provided the use of compounds of formula (I) as defined above in combination with other therapeutically effective compounds used in the treatment of the skin disorders and diseases and other conditions referred to above. Also provided are formulations comprising a compound of formula (I) together with one or more of such other therapeutically effective compounds. Suitable other therapeutically effective compounds include, for example, vitamin A, vitamin C, vitamin E, co-enzyme Q, urea (particularly 1-30%), allantoin (particularly 0.1-1%), benzoyl peroxide (5-10%) menthol, lecithin, salicylic acid (particularly 0.5-10%), panthenol (particularly 0.5-5%), and antibiotics, especially erythromycin base (1-5%), clindamycin phosphate (1-5%) and tetracycline hydrochloride (1-5%), the amounts specified in parentheses being particularly suitable, but non-limiting, amounts by weight for such formulations.

The following non-limiting Examples serve to further illustrate the present invention. The percentage amounts referred to are by weight

### Example 1

| An ointment consisting of: | |
|---|---|
| Eucerine | 30% |
| Glycerol | 60% |
| Compound of formula (I) | 10% |

The ointment was prepared as follows. The compound of formula (I) was powdered and blended with a small amount of Eucerine until homogeneous, then the remaining Eucerine and glycerol were added and the whole mixture stirred until homogeneous.

An ointment of Example 1 containing 1-methylnicotinamide chloride was applied topically for the treatment of psoriasis in a group of 7 patients. After ten days treatment with the ointment six of the patients showed a considerable improvement consisting in flattening of papula and cessation of flares. None of the patients showed any negative effects of the treatment with the ointment.

### Example 2

| A gel consisting of: | |
|---|---|
| Methylcellulose | 5% |
| Glycerol | 12% |
| Water | 82.5% |
| Compound of formula (I) | 0.5% |

The gel was prepared as follows. Powdered methylcellulose was added to hot water while stirring intensively. The resulting dispersion was cooled to about 6°C and blended with glycerol. Compound of formula (I) was then added and the whole mixture stirred until homogeneous.

A gel of Example 2 containing 1-methylnicotinamide citrate was applied for topical treatment of leg ulcers in a group of 5 patients. After ten days treatment all the patients showed a significant improvement consisting in accelerating of granulation and epithelisation. None of the patients showed any negative effects of the treatment with the gel.

### Example 3

| A gel consisting of: | |
|---|---|
| Methylcellulose | 5% |
| Glycerol | 12% |
| Water | 82.7% |
| Compound of formula (I) | 0.3% |

The gel was prepared analogously to Example 3.

A gel of Example 3 containing 1-methylnicotinamide chloride was applied for topical treatment of juvenile acne in a group of 6 patients. After treatment there was cessation of active pathological symptoms in all of the patients.

An analagous gel formulation to Examples 2 and 3 comprising polyacrylic acid, propylene glycol, ethanol, triethanolamine, paraben and demineralised water, in addition to a compound of formula (I), has also been shown to be clinically effective.

### Example 4

| A solution consisting of: | |
|---|---|
| Ethyl alcohol | 40% |
| Water | 59.99% |
| Compound of formula (I) | 0.01% |

The solution was prepared as follows. The compound of formula (I) was dissolved in water, then ethyl alcohol was added and the resulting solution cooled to ambient temperature.

A solution of Example 4 containing 1-methylnicotinamide lactate was applied in order to prevent a recurrence of acne juvenile in the group of patients referred to in Example 3. All the patients showed a prolonged period of improvement after cessation of active pathological symptoms. None of the patients showed any negative effects of the treatment with the solution.

### Example 5

The influence of topical application of 1-methylnicotinamide salts on hair loss was studied in 10 patients with alopecia areata and in 24 patients with hair loss with no particular known cause.

Patients with alopecia areata used a shampoo containing 0.5% by weight of 1-methylnicotinamide chloride either every day or every second day, and applied the gel of Example 3 twice daily. Hair regrowth was observed in 8 of the patients during the first month of treatment. In the remaining patients, although hair regrowth was not observed after one month treatment, the progression of the disease has been stopped.

The other patients with hair loss used the shampoo containing 0.5% by weight of 1-methylnicotinamide chloride daily. In all patients the progression of hair loss has been stopped; in some patients hair regrowth has been observed.

None of the patients reported adverse effects of the treatment, such as itching and skin irritation.

In addition, a surprising effect seen in conjunction with the treatment of hair loss, is that in some cases grey hair was restored to its previous natural colour; in addition, such treatment is thought to prevent hair turning grey. This use of the compounds of formula (I) as defined above represents a further aspect of the present invention.

### Example 6

The influence of topical application of 1-methylnicotinamide salts on skin burns was studied in 19 patients.

The gel of Example 3 was topically applied several times a day directly to the affected area in patients with burns from UV irradiation and high temperature, as well as scalds from hot liquids. There were 15 patients with first degree burns and 4 patients with second degree burns.

In the patients with first degree burns, pain and oedema diminished within the first 24 hours, erythema resolved within 2-3 days in 11 of the patients, and in the remaining 4 patients was resolved after 6 days.

In the patients with second degree burns, the pain also diminished within the first 2 days, blisters were absorbed in 1-3 days, oedema in 2-3 days. The epithelisation process of erosions and superficial ulcerations was very quick and occurred within 7 to 14 days depending on the area.

The residual scars, which were the result of ulcer healing, were considered to be purely cosmetic, and were accepted as such by both patient arid physician. No adverse effects of the treatment were noted.

In addition, similar application of the gel of Example 3 to ten patients with severe sunburn resulted in effective improvement in their condition.

### Further Examples

Similarly, formulations containing the following compounds have been shown to be clinically effective in treating the skin diseases and disorders referred to above, particularly acnes and psoriasis:
1-methylnicotinic acid;
1-methylnicotinic acid ethyl ester salts;
1-methylnicotinic acid propyl ester salts.

Similarly, formulations containing 1-methyl-N'-hydroxymethylnicotinamide salts have been shown to be clinically effective in treating the skin diseases and disorders referred to above.

## Claims

1. The use of a compound of formula (I): 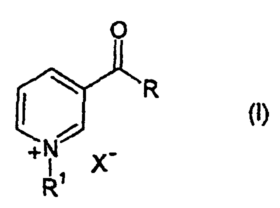 wherein R represents the group NR²R³ or the group OR⁴;
R¹ represents methyl;
R² and R⁴ each independently represent hydrogen or C₁₋₄alkyl;
R³ represents hydrogen, C₁₋₄alkyl or CH₂OH;
and X⁻ is a physiologically suitable counter-anion;
in the preparation of a medicament for the treatment of skin diseases or disorders.

2. The use of a compound of formula (I) as defined in claim 1 in the preparation of a medicament for the treatment of sunburn, burns, scalds and for wound healing.

3. The use as claimed in claim 1 or claim 2 in which R represents the group NR²R³.

4. The use as claimed in any one of claims 1 to 3 in which R² represents methyl or hydrogen.

5. The use as claimed in any one of claims 1 to 4 in which R³ represents CH₂OH or hydrogen.

6. The use as claimed in claim 1 or claim 2 in which R represents the group OR⁴, and R⁴ represents C₁₋₄ alkyl.

7. The use as claimed in any one of claims 1, 2 or 6 in which R⁴ represents propyl or ethyl.

8. The use as claimed in any one of claims 1 to 7 wherein the compound of formula (I) is selected from:
a 1-methylnicotinamide salt; or
a 1-methyl-N'-hydroxymethylnicotinamide salt.

9. The use as claimed in any one of claims 1 to 7 wherein the compound of formula (I) is selected from:
a 1-methylnicotinic acid ethyl ester salt; or
a 1-methylnicotinic acid propyl ester salt.

10. The use as claimed in any one of claims 1 to 7 wherein the compound of formula (I) is selected from:
a 1-methylnicotinic acid salt.

11. The use as claimed in any one of claims 1 to 10 wherein the salt is a chloride, benzoate, salicylate, acetate, citrate or lactate.

12. The use as claimed in claim 1 or claim 2 wherein the compound of formula (I) is selected from:
1-methylnicotinamide chloride;
1-methylnicotinamide citrate;
1-methylnicotinamide lactate;
1-methyl-N'-hydroxymethylnicotinamide chloride;
1-methylnicotinic acid chloride;
1-methylnicotinic acid ethyl ester chloride; or
1-methylnicotinic acid propyl ester chloride.

13. A compound of formula (I): 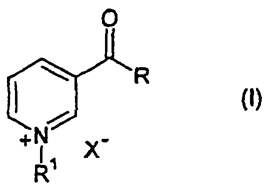 wherein R represents the group NR²R³ or the group OR⁴;
R¹ represents methyl;
R² represents hydrogen or C₁₋₄ alkyl;
R³ represents hydrogen, C₁₋₄ alkyl or CH₂OH;
R⁴ represents C₁₋₄ alkyl;
and X⁻ is a physiologically suitable counter-anion;
for use in therapy.

14. A compound of formula (I) as defined in any one of claims 3 to 9 and 11 for use in therapy.

15. A compound of formula (I) as defined in claim 15, wherein the compound is selected from:
1-methylnicotinamide chloride;
1-methylnicotinamide citrate;
1-methylnicotinamide lactate;
1-methyl-N'-hydroxymethylnicotinamide chloride;
1-methylnicotinic acid ethyl ester chloride;
1-methylnicotinic acid propyl ester chloride;
for use in therapy.

16. A pharmaceutical formulation comprising a compound of formula (I) as defined in any one of claims 15 to 17 together with one or more pharmaceutically acceptable carriers, diluents or excipients.

17. A formulation as claimed in daim 16, which is for topical administration and comprises about 90 to 99,95% of a pharmaceutical base carrier and about 0.005 to about 10% by weight of the compound of formula (I).

18. A formulation as claimed in claim 17 containing about 0.01 to about 10% by weight of the compound of formula (I).

19. A formulation as claimed in daim 17 or claim 18 in the form of an ointment or gel.

20. A formulation as claimed in claim 17 or claim 18 in the form of an aqueous solution.

21. A compound of formula (I) as claimed in any one of claims 1-12, for use in cosmetology.

22. A compound of formula (I) as defined in claim 21, wherein the compound is selected from:
1-methylnicotinamide chloride;
1-methylnicotinamide citrate;
1-methylnicotinamide lactate;
1-methyl-N'-hydroxymethylnicotinamide chloride;
1-methylnicotinic acid ethyl ester chloride;
1-methylnicotinic acid propyl ester chloride;
for use in cosmetology.

23. A method of cosmetic treatment of a skin of a human or animal subject, comprising the administartion to said subject of an effective amount of a compound of formula (I) as defined in any one of claims 1 to 12.

## Patentansprüche

1. Verwendung einer Verbindung der Formel (I): 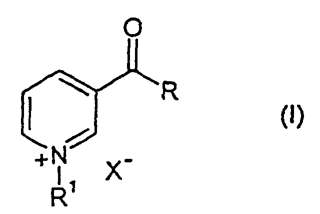 worin R die Gruppe NR²R³ oder die Gruppe OR⁴ bedeutet;
R¹ einen Methylrest bedeutet;
R² und R⁴ unabhängig voneinander jeweils Wasserstoff oder einen C₁₋₄-Alkylrest bedeuten;
R³ Wasserstoff, einen C₁₋₄-Alkylrest oder CH₂OH bedeutet
und X⁻ ein physiologisch geeignetes Gegenanion bedeutet;
für die Herstellung eines Medikaments zur Behandlung von Hautkrankheiten oder Hautstörungen.

2. Verwendung einer Verbindung der Formel (I) gemäß Anspruch 1 zur Herstellung eines Medikaments für die Behandlung von Sonnenbrand, Verbrennungen oder Verbrühungen sowie zur Wundheilung.

3. Verwendung gemäß Anspruch 1 oder 2, wobei R die Gruppe NR²R³ bedeutet.

4. Verwendung gemäß einem der Ansprüche 1 bis 3, wobei R² eine Methylgruppe oder Wasserstoff bedeutet.

5. Verwendung gemäß einem der Ansprüche 1 bis 4, wobei R³ CH₂OH oder Wasserstoff bedeutet.

6. Verwendung gemäß Anspruch 1 oder 2, wobei R die Gruppe OR⁴ und R⁴ einen C₁₋₄-Alkylrest bedeuten.

7. Verwendung gemäß einem der Ansprüche 1, 2 oder 6, wobei R⁴ eine Propyl- oder Ethylgruppe bedeutet.

8. Verwendung gemäß einem der Ansprüche 1 bis 7, wobei die Verbindung der Formel (I) ein Salz von 1-Methylnicotinamid oder ein Salz von 1-Methyl-N'-hydroxymethylnicotinamid ist.

9. Verwendung gemäß einem der Ansprüche 1 bis 7, wobei die Verbindung der Formel (I) ein Salz von 1-Methylnicotinsäureethylester oder ein Salz von 1-Methylnicotinsäurepropylester ist.

10. Verwendung gemäß einem der Ansprüche 1 bis 7, wobei die Verbindung der Formel (I) ein Salz der 1-Methylnicotinsäure ist.

11. Verwendung gemäß einem der Ansprüche 1 bis 10, wobei das Salz ein Chlorid, Benzoat, Salicylat, Acetat, Citrat oder Lactat ist.

12. Verwendung gemäß Anspruch 1 oder 2, wobei die Verbindung der Formel (I)
1-Methylnicotinamid-chlorid;
1-Methylnicotinamid-citrat;
1-Methylnicotinamid-lactat;
1-Methyl-N'-hydroxymethylnicotinamid-chlorid;
1-Methylnicotinsäure-chlorid;
1-Methylnicotinsäure-ethylester-chlorid oder
1-Methylnicotinsäure-propylester-chlorid ist.

13. Verbindung der Formel (I) 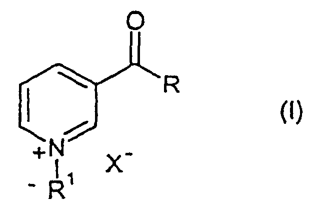 worin R eine NR²R³-Gruppe oder eine OR⁴-Gruppe bedeutet;
R¹ eine Methylgruppe bedeutet;
R² Wasserstoff oder eine C₁₋₄-Alkylgruppe bedeutet;
R³ Wasserstoff, eine C₁₋₄-Alkylgruppe oder eine CH₂OH-Gruppe bedeutet;
R⁴ eine C₁₋₄-Alkylgruppe bedeutet und
X⁻ ein physiologisch geeignetes Gegenanion bedeutet;
zur Verwendung zur Therapie.

14. Verbindung der Formel (I) gemäß einem der Ansprüche 3 bis 9 und 11 zur Verwendung zur Therapie.

15. Verbindung der Formel (I) gemäß Anspruch 13, wobei die Verbindung
1-Methylnicotinamid-chlorid;
1-Methylnicotinamid-citrat;
1-Methylnicotinamid-lactat;
1-Methyl-N'-hydroxymethylnicotinamid-chlorid;
1-Methylnicotinsäure-ethylester-chlorid oder
1-Methylnicotinsäure-propylester-chlorid ist, zur Verwendung zur Therapie.

16. Pharmazeutische Zusammensetzung, enthaltend eine Verbindung der Formel (I) gemäß einem der Ansprüche 13 bis 15 in Verbindung mit einem oder mehreren pharmazeutisch annehmbaren Trägern, Verdünnungsmitteln oder Hilfsmitteln.

17. Zusammensetzung gemäß Anspruch 16, zur topischen Anwendung und enthaltend etwa 90 bis 99,95% eines pharmazeutischen Grundträgers und etwa 0,005 bis etwa 10 Gewichts-% an der Verbindung gemäß Formel (I).

18. Zusammensetzung gemäß Anspruch 17, enthaltend etwa 0,01 bis etwa 10 Gewichts-% der Verbindung gemäß Formel (I).

19. Zusammensetzung gemäß Anspruch 17 oder 18 in Form einer Salbe oder eines Gels.

20. Zusammensetzung gemäß Anspruch 17 oder 18 in Form einer wässerigen Lösung.

21. Verbindung der Formel (I) gemäß einem der Ansprüche 1 bis 12 zur Verwendung als Kosmetikum.

22. Verbindung der Formel (I) gemäß Anspruch 21 aus der Gruppe, bestehend aus
1-Methylnicotinamid-chlorid;
1-Methylnicotinamid-citrat;
1-Methylnicotinamid-lactat;
1-Methyl-N'-hydroxymethylnicotinamid-chlorid;
1-Methylnicotinsäure-ethylester-chlorid oder
1-Methylnicotinsäure-propylester-chlorid, zur Verwendung als Kosmetikum.

23. Verfahren zur kosmetischen Behandlung der Haut eines Menschen oder Tieres, wobei man den Menschen oder das Tier mit einer wirksamen Menge einer Verbindung der Formel (I) gemäß einem der Ansprüche 1 bis 12 behandelt.

## Revendications

1. Utilisation d'un composé de formule (I): 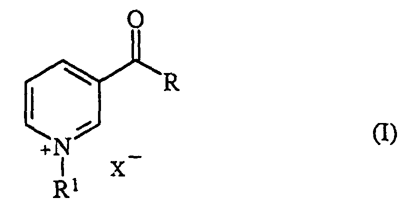 dans laquelle
R représente un groupe de formule NR²R³ ou OR⁴,
R¹ représente un groupe méthyle,
R² et R⁴ représentent chacun, indépendamment, un atome d'hydrogène
ou un groupe alkyle en C₁₋₄,
R³ représente un atome d'hydrogène, un groupe alkyle en C₁₋₄, ou un groupe hydroxyméthyle CH₂OH,
et X⁻ représente un contre-ion physiologiquement approprié; dans la préparation d'un
médicament destiné au traitement des troubles ou des maladies de la peau.

2. Utilisation d'un composé de formule (I), tel que défini dans la revendication 1, dans la préparation d'un médicament servant au traitement des brûlures par le soleil, des brûlures et des échaudures, ou à la cicatrisation de blessures.

3. Utilisation, selon la revendication 1 ou 2, dans lequelle R représente un groupe de formule NR²R³.

4. Utilisation selon l'une quelconque des revendications 1 à 3, dans lequelle R² représente un groupe méthyle ou un atome d'hydrogène.

5. Utilisation selon l'une quelconque des revendications 1 à 4, dans lequelle R³ représente un groupe hydroxyméthyle ou un atome d'hydrogène.

6. Utilisation selon la revendication 1 ou 2, dans lequelle R représente un groupe de formule OR⁴ où R⁴ représente un groupe alkyle en C₁₋₄.

7. Utilisation selon l'une quelconque des revendications 1, 2 et 6, dans lequelle R⁴ représente un groupe propyle ou éthyle.

8. Utilisation selon l'une quelconque des revendications 1 à 7, dans lequelle le composé de formule (I) est un sel du 1-méthyl-nicotinamide ou un sel du 1-méthyl-N'-hydroxyméthyl-nicotinamide.

9. Utilisation selon l'une quelconque des revendications 1 à 7, dans lequelle le composé de formule (I) est un sel de 1-méthyl-nicotinate d'éthyle ou un sel de 1-méthyl-nicotinate de propyle.

10. Utilisation selon l'une quelconque des revendications 1 à 7, dans lequelle le composé de formule (I) est un sel de l'acide 1-méthyl-nicotinique.

11. Utilisation selon l'une quelconque des revendications 1 à 10, dans lequelle le sel est un chlorure, un benzoate, un salicylate, un acétate, un citrate ou un lactate.

12. Utilisation selon la revendication 1 ou 2, dans lequelle le composé de formule (I) est choisi parmi les suivants:
chlorure de 1-méthyl-nicotinamide;
citrate de 1-méthyl-nicotinamide;
lactate de 1-méthyl-nicotinamide;
chlorure de 1-méthyl-N'-hydroxyméthyl-nicotinamide;
chlorure d'acide 1-méthyl-nicotinique;
chlorure de 1-méthyl-nicotinate d'éthyle;
et chlorure de 1-méthyl-nicotinate de propyle.

13. Composé de formule (I) : 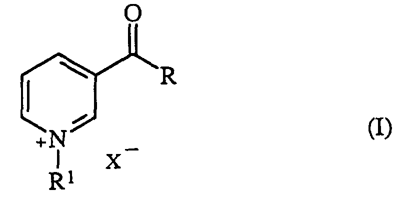 dans laquelle
R représente un groupe de formule NR²R³ ou OR⁴,
R¹ représente un groupe méthyle,
R² représente un atome d'hydrogène ou un groupe alkyle en C₁₋₄,
R³ représente un atome d'hydrogène, un groupe alkyle en C₁₋₄, ou un groupe hydroxyméthyle CH₂OH,
R⁴ représente un groupe alkyle en C₁₋₄,
et X⁻ représente un contre-ion physiologiquement admissible, pour une utilisation en therapie.

14. Composé de formule (I), tel que défini dans l'une quelconque des revendications 3 à 9 et 11, pour utilisation en therapie.

15. Composé de formule (I), tel que défini dans la revendication 13, lequel composé est choisi parmi les suivants:
chlorure de 1-méthyl-nicotinamide ;
citrate de 1-méthyl-nicotinamide ;
lactate de 1-méthyl-nicotinamide ;
chlorure de 1-méthyl-N'-hydroxyméthyl-nicotinamide
chlorure de 1-méthyl-nicotinate d'éthyle ;
et chlorure de 1-méthyl-nicotinate de propyle ;
pour utilisation en therapie.

16. Formulation pharmaceutique comprenant un composé de formule (I), tel que défini dans l'une des revendications 13 à 15, ainsi qu'un ou plusieurs véhicules, diluants ou excipients pharmacologiquement acceptables.

17. Formulation selon la revendication 16, etant pour administration topique et comprenant d'environ de 90 à 99,95 % d'un véhicule-base pharmaceutique et d'environ de 0,005 à 10 % en poids de composé de formule (I).

18. Formulation selon la revendication 17, contenant d'environ de 0,01 à 10 % en poids de composé de formule (I).

19. Formulation selon la revendication 17 ou 18, se présentant sous forme de pommade ou de gel.

20. Formulation selon la revendication 17 ou 18, se présentant sous forme de solution aqueuse.

21. Composé de formule (I), tel que défini dans l'une quelconque des revendications 1 à 12, pour utilisation en cosmétique.

22. Composé de formule (I), selon la revendication 21, lequel composé est choisi parmi les suivants:
chlorure de 1-méthyl-nicotinamide;
citrate de 1-méthyl-nicotinamide;
lactate de 1-méthyl-nicotinamide;
chlorure de 1-méthyl-N'-hydroxyméthyl-nicotinamide;
chlorure de 1-méthyl-nicotinate d'éthyle ;
et chlorure de 1-méthyl-nicotinate de propyle ;
pour utilisation en cosmétique.

23. Procédé de traitement cosmétique de la peau d'un sujet humain ou animal, comprenant l'administration audit sujet une quantité efficace d'un composé de formule (I), tel que défini dans l'une quelconque des revendications 1 à 12.
